(19) Europäisches Patentamt
European Patent Office
Office européen des brevets

(11) Veröffentlichungsnummer: **0 062 864**
**B1**

(12) # EUROPÄISCHE PATENTSCHRIFT

(45) Veröffentlichungstag der Patentschrift: **24.07.85**

(51) Int. Cl.⁴: **C 07 D 249/16**, C 07 D 249/18

(21) Anmeldenummer: **82102854.5**

(22) Anmeldetag: **03.04.82**

(54) **Verfahren zur Herstellung von kondensierten 1,2,3-Triazolen.**

(30) Priorität: **14.04.81 DE 3115070**

(43) Veröffentlichungstag der Anmeldung: **20.10.82 Patentblatt 82/42**

(45) Bekanntmachung des Hinweises auf die Patenterteilung: **24.07.85 Patentblatt 85/30**

(84) Benannte Vertragsstaaten: **BE CH DE FR GB LI**

(56) Entgegenhaltungen:
**DE - A - 2 822 506**
**US - A - 3 413 227**
**US - A - 3 639 431**

(73) Patentinhaber: **BAYER AG, Konzernverwaltung RP Patentabteilung, D-5090 Leverkusen 1 Bayerwerk (DE)**

(72) Erfinder: **Hagedorn, Ferdinand, Dr., Domblick 16, D-5090 Leverkusen 31 (DE)**
Erfinder: **Evertz, Werner, Wolffstrasse 1, D-4019 Monheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von kondensierten 1,2,3-Triazolen durch Umsetzung von aromatischen Diaminoverbindungen mit einem Ester der salpetrigen Säure.

Aus der DE-OS 2 822 506 ist bekannt, kondensierte Triazole durch Umsetzung eines o-Diamins aus der Reihe der aromatischen 6-Ringe mit einem Alkylnitrit in Gegenwart einer Mineralsäure oder einer Carbonsäure als Katalysator in einem organischen Lösungsmittel herzustellen. Die als Katalysatoren eingesetzten Säuren müssen nach Beendigung der Umsetzung neutralisiert und abgetrennt werden.

Es wurde nun ein Verfahren zur Herstellung von kondensierten 1,2,3-Triazolen durch Umsetzung von ortho-Diaminoarylen mit einem Ester der salpetrigen Säure in Gegenwart eines Katalysators in einem Lösungs- oder Verdünnungsmittel gefunden, das dadurch gekennzeichnet ist, dass man als Katalysator das herzustellende 1,2,3-Triazol zusetzt.

Als Reaktionsprodukte des erfindungsgemässen Verfahrens, die erfindungsgemäss als Katalysatoren eingesetzt werden, seien 1,2,3-Triazole der Formel

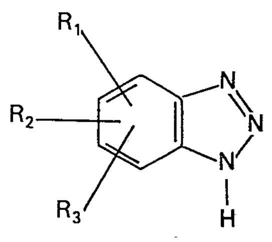

(I),

in der

$R^1$, $R^2$ und $R^3$ gleich oder verschieden sind und Wasserstoff, Alkyl, Aryl, Aralkyl, Halogen, Alkoxy oder Nitro bedeuten und wobei zwei benachbarte Kohlenwasserstoffreste zu einem aromatischen 5- oder 6-Ring verbunden sein können, genannt.

Alkyl kann erfindungsgemäss ein geradkettiger oder verzweigter Kohlenwasserstoffrest mit 1 bis 12 Kohlenstoffatomen sein. Bevorzugtes Alkyl ist Niederalkyl mit 1 bis etwa 6 Kohlenstoffatomen. Beispielsweise seien die folgenden Alkylreste genannt: Methyl, Ethyl, Propyl, Isopropyl, Butyl, Isobutyl, Pentyl, Isopentyl, Hexyl und Isohexyl.

Aryl kann erfindungsgemäss ein aromatischer Kohlenwasserstoffrest mit 6 bis 12 Kohlenstoffatomen sein. Ein bevorzugtes Aryl ist der Phenylrest.

Aralkyl kann erfindungsgemäss im aliphatischen Teil ein geradkettiger oder verzweigter Kohlenwasserstoffrest mit 1 bis 6 Kohlenstoffatomen und im aromatischen Teil ein aromatischer Kohlenwasserstoffrest, bevorzugt der Phenyl- oder Naphthylrest, sein. Bevorzugte Aralkylreste sind der Benzyl- und der α- oder β-Methylennaphthylrest.

Halogen kann erfindungsgemäss Fluor, Chlor, Brom oder Jod, bevorzugt Chlor oder Brom, sein.

Alkoxy kann erfindungsgemäss im aliphatischen Teil ein geradkettiger oder verzweigter, aliphatischer Kohlenwasserstoffrest mit 1 bis 12 Kohlenstoffatomen sein. Bevorzugtes Alkoxy ist der Niederalkoxyrest mit 1 bis etwa 6 Kohlenstoffatomen. Beispielsweise seien die folgenden Alkoxyreste genannt: Methoxy, Ethoxy, Propoxy, Isopropoxy, Butoxy, Isobutoxy, Pentoxy, Isopentoxy, Hexoxy und Isohexoxy.

Bevorzugte 1,2,3-Triazole sind Verbindungen der Formel

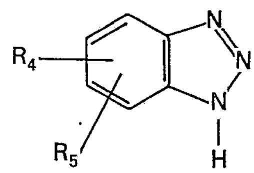

(II),

in der

$R^4$ und $R^5$ gleich oder verschieden sind und Wasserstoff, Niederalkyl, Phenyl, Chlor, Brom, Niederalkoxy oder Nitro bedeuten.

Beispielsweise seien die folgenden 1,2,3-Triazole genannt: Benzotriazol, 5-Methyl-benzotriazol, 4-Methyl-benzotriazol, Gemische von 5- und 4-Methyl-benzotriazol, Gemische von Benzotriazol und Methyl-benzotriazolen, 1,2-Naphthotriazol und 2,3-Naphthotriazol.

Für das erfindungsgemässe Verfahren wird das 1,2,3-Triazol als Katalysator im allgemeinen in einer Menge von 0,001 bis 0,5 Mol, bevorzugt 0,01 bis 0,1 Mol, pro Mol des ortho-Diaminoarylens eingesetzt.

Ortho-Diaminoaryle für das erfindungsgemässe Verfahren können beispielsweise Verbindungen der Formel

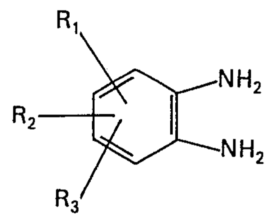

(III),

in der

$R^1$, $R^2$ und $R^3$ die obengenannte Bedeutung haben, sein.

Bevorzugte ortho-Diaminoaryle für das erfindungsgemässe Verfahren sind Verbindungen der Formel

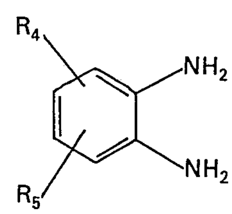

(IV),

in der

$R^4$ und $R^5$ die obengenannte Bedeutung haben.

Beispielsweise seien die folgenden ortho-Diaminoaryle genannt: 1,2-Diaminobenzol, 2,3-Diamino-toluol, 3,4-Diaminotoluol, 1,2-Diaminonaphthalin und 2,3-Diaminonaphthalin. Bevorzugte ortho-Diaminoaryle für das erfindungsgemässe Verfahren sind: 1,2-Diaminobenzol, 2,3-Diaminotoluol und 3,4-Diaminotoluol.

Es ist selbstverständlich auch möglich, Gemische der ortho-Diaminoaryle einzusetzen. So kann man beispielsweise vorteilhafterweise ein Isomerengemisch aus 2,3- und 3,4-Diaminotoluol einsetzen.

Die erfindungsgemäss zu verwendenden ortho-Diamine sind an sich bekannt und können nach bekannten Methoden hergestellt werden.

Ester der salpetrigen Säure für das erfindungsgemässe Verfahren sind im allgemeinen die für Diazotierungsreaktionen übliche Ester der salpetrigen Säure mit primären oder sekundären Alkoholen (Houben-Weyl, Band X/3, Seiten 28 und 29 [1965]). Für das erfindungsgemässe Verfahren werden Ester der salpetrigen Säure mit niederen primären Alkoholen (1 bis etwa 6 Kohlenstoffatomen) bevorzugt. Als Ester der salpetrigen Säure seien beispielsweise Methylnitrit, Ethylnitrit, Propylnitrit, Isopropylnitrit, Butylnitrit, Isobutylnitrit, Pentylnitrit, Isopentylnitrit, Hexylnitrit und Isohexylnitrit genannt. Für das erfindungsgemässe Verfahren wird Methylnitrit bevorzugt.

Die Herstellung der Ester der salpetrigen Säure ist an sich bekannt.

Für das erfindungsgemässe Verfahren setzt man den Ester der salpetrigen Säure im alllgemeinen in einer Menge von 1,0 bis 1,2 Mol, bevorzugt von 1,0 bis 1,1 Mol, pro Mol des ortho-Diaminoarylens ein.

Das erfindungsgemässe Verfahren wird im allgemeinen im Temperaturbereich von −20 bis 100°C, bevorzugt von 20 bis 80°C, durchgeführt. Im allgemeinen führt man das erfindungsgemässe Verfahren bei Normaldruck durch. Es ist selbstverständlich jedoch auch möglich, das Verfahren bei einem Unterdruck oder einem Überdruck durchzuführen.

Das erfindungsgemässe Verfahren wird in Lösungs- oder Verdünnungsmitteln durchgeführt, die sich unter den Reaktionsbedingungen nicht verändern. Es ist möglich, für das erfindungsgemässe Verfahren protische oder aprotische Lösungsmittel einzusetzen.

Insbesondere seien genannt: Alkohole, Glykole, Glykolether, aromatische Kohlenwasserstoffe und chlorierte aromatische Kohlenwasserstoffe. Es ist auch möglich, dass die Lösungs- oder Verdünnungsmittel für das erfindungsgemässe Verfahren Wasser, im allgemeinen bis zu max. 40%, enthalten. Bevorzugte Lösungs- oder Verdünnungsmittel für das erfindungsgemässe Verfahren sind Methanol, wasserhaltiges Methanol, Ethanol, Isopropanol, Toluol, Chlorbenzol, o-Dichlorbenzol und Xylol.

Das erfindungsgemässe Verfahren kann beispielsweise wie folgt durchgeführt werden:

Die als Katalysatoren eingesetzten kondensierten 1,2,3-Triazole werden vor Beginn der Umsetzung dem Reaktionsgemisch zugegeben, z.B. in Substanz oder gelöst in einem Lösungsmittel oder in dem jeweils umzusetzenden ortho-Diaminoaryl bzw. in dem ortho-Diaminoaryl-Gemisch.

Nach beendeter Umsetzung kann das kondensierte 1,2,3-Triazol nach bekannten Methoden, z.B. durch Destillieren, isoliert werden.

Die Verwendung des jeweiligen Reaktionsendproduktes als Katalysator für die Diazotierungs-Ringschlussreaktion vereinfacht die Herstellung der kondensierten 1,2,3-Triazole und erleichtert deren Aufarbeitung und Isolierung. Die nach dem bekannten Verfahren notwendige Abtrennung und Beseitigung der als Katalysatoren verwendeten Säuren bzw. deren Salze entfällt vorteilhafterweise bei dem erfindungsgemässen Verfahren. Die nach dem erfindungsgemässen Verfahren hergestellten kondensierten 1,2,3-Triazole können als Korrosionsinhibitoren verwendet werden (US-PS 2 861 078, DE-OS 2 877 188 und DE-AS 1 285 835).

Beispiel 1

In eine Suspension von 108,1 Gew.-Teilen o-Phenylendiamin und 3 Gew.-Teilen Benzotriazol in 400 Vol.-Teilen Xylol-Isomerengemisch unter Stickstoff-Schutzgas leitet man innerhalb 2 Stunden bei 20 bis 25°C gasförmiges Methylnitrit ein (hergestellt aus 72 Gew.-Teilen Natriumnitrit, 43 Vol.-Teilen Wasser, 45 Vol.-Teilen Methanol und Zutropfen einer Schwefelsäure, hergestellt aus 80 Gew.-Teilen Eis und 40 Vol.-Teilen konz. Schwefelsäure, 96%ig). Das Gemisch wird 30 Minuten nachgerührt, anschliessend unter Rühren 1 Stunde bei 50°C erhitzt. Nach Stehen über Nacht werden Methanol, Wasser und 120 Vol.-Teile Xylol abdestilliert. Man gibt 370 Vol.-Teile Xylol zu und saugt nach Erstarren das kristalline ausgeschiedene Benzotriazol ab. Durch Wiederverwenden der Mutterlauge erhält man Benzotriazol in fast quantitativer Ausbeute mit einem Gehalt von 99%.

Beispiel 2

In einem Gemisch von 351 Gew.-Teilen 2,3- und 3,4-Diaminotoluol im Verhältnis etwa 40:60, 500 Vol.-Teilen Methanol und 18 Gew.-Teilen eines Isomerengemisches von 4- und 5-Methylbenzotriazol im Verhältnis etwa 40:60 leitet man unter Rühren und Kühlen bei 45 bis 50°C gasförmiges Methylnitrit ein bis zur vollständigen Umsetzung des Diamin-Isomerengemisches. Das Reaktionsgemisch wird gegen Ende der Umsetzung ca. 40 Minuten auf Rückflusstemperatur erhitzt, anschliessend im Vakuum von Methanol, überschüssigem Methylnitrit und Wasser befreit und durch Dünnschichtdestillation gereinigt. Ausbeute: 96% der Theorie.

Beispiel 3

In eine Lösung von 118 Gew.-Teilen 3,4-Diamino-toluol, 3 Gew.-Teilen 5-Methyl-benzotriazol, gelöst in 5 Vol.-Teilen Methanol, in 250 Vol.-Tei-

len o-Dichlorbenzol unter Stickstoff-Schutzgas leitet man innerhalb 2 Stunden bei 40 bis 45°C gasförmiges Methylnitrit ein (hergestellt aus 72 Gew.-Teilen Natriumnitrit, 43 Vol.-Teilen Wasser, 45 Vol.-Teilen Methanol und Zutropfen einer Mischung aus 80 Gew.-Teilen Eis und 40 Vol.-Teilen konz. Schwefelsäure, 96%ig). Das Gemisch wird 1 Stunde bei 40 bis 45°C, anschliessend 30 Minuten bei 80 bis 85°C unter Rühren nacherhitzt.

Nach Entfernen von überschüssigem Methylnitrit und Abdestillieren von Methanol, Wasser und der Hauptmenge des Lösungsmittels rührt man den Rückstand mit 200 Vol.-Teilen Cyclohexan 15 Minuten am Rückfluss, kühlt das Gemisch, saugt das kristalline Produkt ab, wäscht es mit 100 Vol.-Teilen Cyclohexan und trocknet es. Ausbeute: fast quantitativ, Gehalt: 99,5% (GC).

Beispiel 4

Entsprechend der Vorschrift des Beispiels 3 werden 118 Gew.-Teile 2,3-Diamino-toluol mit Methylnitrit in o-Dichlorbenzol umgesetzt, wobei in diesem Falle 3 Gew.-Teile 4-Methyl-benzotriazol als Katalysator vorgelegt werden. Nach der Aufarbeitung erhält man 129 Gew.-Teile 99,8%iges 4-Methyl-benzotriazol, Fp. 149 bis 150°C.

## Patentansprüche

1. Verfahren zur Herstellung von kondensierten 1,2,3-Triazolen durch Umsetzung von ortho-Diaminoarylen mit einem Ester der salpetrigen Säure in Gegenwart eines Katalysators in einem Lösungs- oder Verdünnungsmittel, dadurch gekennzeichnet, dass man als Katalysator das herzustellende kondensierte 1,2,3-Triazol zusetzt.

2. Verfahren nach Anspruch 1, dadurch gekennzeichnet, dass man den Katalysator in einer Menge von 0,001 bis 0,5 Mol pro Mol des eingesetzten ortho-Diaminoaryls einsetzt.

## Claims

1. Process for the preparation of fused 1,2,3-triazoles by reacting ortho-diaminoaryls with an ester of nitrous acid in the presence of a catalyst in a solvent or diluent, characterised in that the fused 1,2,3-triazole to be prepared is added as the catalyst.

2. Process according to Claim 1, characterised in that the catalyst is used in a quantity of 0.001 to 0.5 mol per mol of the ortho-diaminoaryl used.

## Revendications

1. Procédé de fabrication de 1,2,3-triazols condensés par réaction d'ortho-diaminoaryles avec un ester d'acide nitreux en présence d'un catalyseur dans un solvant ou un diluant, caractérisé en ce qu'on ajoute comme catalyseur le 1,2,3-triazol condensé qui doit être préparé.

2. Procédé selon la revendication 1, caractérisé en ce qu'on utilise le catalyseur en une quantité de 0,001 à 0,5 mole par mole de l'ortho-diaminoaryle employé.